# EUROPÄISCHE PATENTSCHRIFT

(11) **EP 0 707 628 B1**
(45) Veröffentlichungstag und Bekanntmachung des Hinweises auf die Patenterteilung: **10.09.1997**
(21) Anmeldenummer: 94921627.9
(22) Anmeldetag: 27.06.1994
(51) Int. Cl.: C11D 3/386, C11D 17/00, C12N 9/98

(54) **UMHÜLLTE ENZYMZUBEREITUNG FÜR WASCH- UND REINIGUNGSMITTEL**
COATED ENZYME COMPOSITION FOR WASHING AND CLEANING PRODUCTS
COMPOSITION ENZYMATIQUE ENROBEE POUR PRODUITS DE LAVAGE ET DE NETTOYAGE

(30) Priorität: 05.07.1993 DE 4322229
(43) Veröffentlichungstag der Anmeldung: 24.04.1996
(73) Patentinhaber: Henkel Kommanditgesellschaft auf Aktien, 40191 Düsseldorf (DE)
(72) Erfinder: PAATZ, Kathleen, D-40589 Düsseldorf (DE); RÄHSE, Wilfried, D-40598 Düsseldorf (DE); PICHLER, Werner, A-6250 Kundl (AT); KÜHNE, Norbert, D-42781 Haan (DE); UPADEK, Horst, D-40833 Ratingen (DE)
(86) Internationale Anmeldenummer: EP9402079
(87) Internationale Veröffentlichungsnummer: WO9502031

(56) Entgegenhaltungen:
- EP-A- 0 206 418
- WO-A-90/09428
- WO-A-91/06638
- WO-A-92/11347
- WO-A-93/07260

## Beschreibung

Die Erfindung betriffl ein Enzymgranulat, ein Verfahren zu seiner Herstellung und die Verwendung des Granulats in festen Wasch- und Reinigungsmitteln.

Enzyme, insbesondere Proteasen, finden ausgedehnte Verwendung in Wasch-, Waschhilfsund Reinigungsmitteln. Üblicherweise kommen die Enzyme dabei nicht als Konzentrate, sondern in Mischungen mit einem Verdünnungs- und Trägermaterial zum Einsatz. Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DT 16 17 190 beziehungsweise durch Aufkleben mit nichtionischen Tensiden gemäß der deutschen Offenlegungsschrift DT 16 17 188 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Marumerisieren in die gewünschte Partikelform überführt, wie zum Beispiel in der deutschen Patentschrift DE 16 17 232, der deutschen Offenlegungsschrift DT 20 32 768, und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im Waschgut verfangen und dieses verunreinigen bzw. sie werden ungenutzt in das Abwasser überführt. Aus der deutschen Offenlegungsschrift DT 18 03 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzten, das entstandene Gemisch zu granulieren und gegebenenfalls das Granulat mit filmbildenden Polymeren und Farbstoffen zu umhüllen. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind. Außerdem weisen die derart hergestellten Granulate eine so hohe Lösungs- beziehungsweise Zerfallsgeschwindigkeit unter Waschbedingungen auf, daß die Granulate teilweise schon bei der Lagerung relativ rasch zerfallen und die Enzyme desaktiviert werden.

Aus der internationalen Patentanmeldung WO 92/11347 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch derartige Zuschlagstoffe wird die Enzymverarbeitung ohne größere Aktivitätsverluste möglich.

Aus der internationalen Patentanmeldung WO 93/07263 ist ein Enzyme enthaltendes Granulat bekannt, das aus einem wasserlöslichen oder -dispergierbaren Kern, der mit einem Vinylpolymer überzogen ist, besteht, auf dem sich eine Schicht aus Enzym und Vinylpolymer befindet, wobei das Granulat einen Außenüberzug aus Vinylpolymer aufweist. Der Außenüberzug kann auch Pigmente enthalten. Durch den Mehrschichtenaufbau ist ein derartiges Enzymgranulat jedoch relativ aufwendig in der Herstellung.

In den internationalen Patentanmeldungen WO 91/06638 und WO 93/07260 sind verschiedene Umhüllungsmaterialien für staubfreie Enzymgranulate, welche durch Aufsprühen einer Fermentationsbrühe auf ein hydratisierbares Trägermaterial erzeugt werden, genannt. Unter anderem werden als geeignet Fettsäureester, alkoxylierte Alkohole, Polyvinylalkohol, Polyethylenglykol, Zucker und Stärke aufgeführt. Jeglicher Hinweis auf das nun als besonders zweckmäßig gefundene und einfach zu verarbeitende Umhüllungssystem fehlt. Aus der europäischen Patentanmeldung EP 0 206 418 sind Enzymgranulate bekannt, die stabil in bleichmittelhaltige Zubereitungen eingearbeitet werden können und die eine Umhüllung aus wasserlöslichem nichtionischem Wachs mit einem Schmelzpunkt von mindestens 38°C aufweisen.

Die in den genannten Dokumenten eingesetzten Überzugsmassen für die äußerste Umhüllungsschicht werden normalerweise in Form einer wäßrigen Dispersion in einem Wirbelschichttrockner auf das Enzymgranulat aufgebracht. Dabei besteht die Gefahr der zumindest oberflächlichen Zerstörung der Granulate durch Staubabrieb in der Wirbelschicht, was zu einem erhöhtem Anteil an äußerst feinkörnigem Material im Enzymgranulat führt, welches für die Zumischung zu üblichen partikelförmigen Wasch- oder Reinigungsmitteln unbrauchbar ist, da es sich nicht homogen in der entstehenden Mischung verteilt. Daher ist man bestrebt, den Feinkornanteil im Enzymgranulat so gering wie möglich zu halten, um möglichst wenig durch Aussieben oder Windsichten entfernen zu müssen.

Es bestand daher die Aufgabe, ein Umhüllungssystem zu entwickeln, das bei gleichmäßigem Auftrag auf ein enzymhaltiges Granulat der oberflächlichen Zerstörung des Granulats entgegenwirkt, die Lagerstabilität des Enzyms durch schützende Umhüllung des gesamten Granulats erhöht, die Überdeckung eventueller Eigenfärbung des nicht umhüllten Enzymgranulats erlaubt und eventuell störenden Geruch des nicht umhüllten Granulats, wahrscheinlich durch Verhinderung der Diffusion der Geruchsstoffe an die Enzymgranulatoberfläche, beseitigt.

Die Erfindung betriffl ein für die Einarbeitung in insbesondere teilchenförmige Wasch- oder Reinigungsmittel geeignetes Enzymgranulat, enthaltend Enzym und anorganisches und/oder organisches Trägermaterial und eine gleichmäßige äußere pigmenthaltige Umhüllungsschicht, welches dadurch gekennzeichnet ist, daß die äußere Umhüllungsschicht aus einem Umhüllungssystem besteht, welches 30 Gew.-% bis 50 Gew.-% feinteiliges anorganisches Pigment, 45 Gew.-% bis 60 Gew.-% eines bei Raumtemperatur festen Alkohols mit einem Schmelzpunkt im Bereich von 45 °C bis 65 °C, bis zu 15 Gew.-%, insbesondere 5 Gew.-% bis 15 Gew.-% Emulgator für den Alkohol, bis zu 5 Gew.-%, insbesondere 0,2 Gew.-% bis 3 Gew.-% Dispergiermittel für das Pigment und bis zu 3 Gew.-% Wasser enthält.

Weiterhin betrifft die Erfindung ein Verfahren zur Herstellung eines für die Einarbeitung in partikelförmige Wasch- oder Reinigungsmittel geeigneten Enzymgranulates mit einer mittleren Korngröße von 0,8 mm bis 1,2 mm durch Extrudieren eines durch Vermischen einer gegebenenfalls zuvor durch Mikrofiltration von unlöslichen Bestandteilen befreiten, aufkonzentrierten Fermentationsbrühe mit anorganischem und/oder organischem Trägermaterial als Zuschlagstoff entstandenen Enzym-Vorgemischs, gegebenenfalls Sphäronisierung des Extrudats in einem Rondiergerät, Trocknung und Aufbringen einer äußeren Umhüllungsschicht, wobei man in einer Wirbelschicht aus Extrudat eine äußere Umhüllungsschicht eines Überzugssystems aus 30 Gew.-% bis 50 Gew.-% feinteiligem anorganischem Pigment, 45 Gew.-% bis 60 Gew.-% Alkohol mit einem Schmelzpunkt im Bereich von 45 °C bis 65 °C, bis zu 15 Gew.-%, insbesondere 5 Gew.-% bis 15 Gew.-% Emulgator für den Alkohol und bis zu 5 Gew.-%, insbesondere 0,2 Gew.-% bis 3 Gew.-% Dispergiermittel für das Pigment aufbringt.

Die Alkoholkomponente des Überzugssystems ist vorzugsweise ein primärer linearer Alkohol mit 14 bis 22 C-Atomen oder ein Gemisch aus diesen. Zu den genannten Alkoholen gehören insbesondere Myristylakohol, Cetylalkohol, Stearylalkohol, Arachidylalkohol, Behenylalkohol und ein- bis dreifach ungesättigte Alkohole entsprechender Kettenlänge, wobei wesentlich ist, daß die genannte Alkoholkomponente des Überzugssystems einen Schmelzpunkt im Bereich von 45 °C bis 65 °C, insbesondere von 50 °C bis 60 °C aufweist, worunter hier die Temperatur verstanden werden soll, bei der beim Erwärmen 100 % der Alkoholkomponente in flüssiger Form vorliegen. Bei Einsatz von Alkoholgemischen sind auch solche brauchbar, welche geringe Anteile, normalerweise unter 15 Gew.-% bezogen auf Alkoholgemisch, an bei Raumtemperatur flüssigen Anteilen enthalten, solange das gesamte Alkoholgemisch bei Raumtemperatur fest erscheint und einen Erstarrungspunkt im Bereich von 45 °C bis 65 °C, insbesondere von 50 °C bis 60 °C aufweist. Der Erstarrungspunkt ist die Temperatur, bei der beim Abkühlen auf eine Temperatur oberhalb des Schmelzpunkts erwämten Materials eine Verfestigung eintritt. Er kann mit Hilfe eines rotierenden Thermometers nach dem Verfahren der DIN IS0 2207 bestimmt werden.

Geeignete Emulgatoren für die Alkoholkomponente sind Substanzen, welche in der Lage sind, die Alkoholkomponente in Wasser zu emulgieren, so daß eine bei Temperaturen bis zu 95 °C versprühbare Mischung entsteht, und/oder welche es erlauben, das Umhüllungssystem in eine möglichst homogene, bei Temperaturen bis zu 120 °C versprühbare Schmelze zu überführen. Als Anhaltspunkt kann in diesem Zusammenhang dienen, daß Flüssigkeiten mit Viskositäten bis zu etwa 10 000 cPs bei den genannten Temperaturen in der Regel problemlos mittels dafür vorgesehener Vorrichtungen versprüht und auf Enzymgranulate aufgebracht werden können. Zu den Emulgatoren für die Alkoholkomponente des Überzugssystems gehören beispielsweise die Ethoxylierungsprodukte der genannten Alkohole, wobei deren Umsetzungsprodukte mit durchschnittlich 25 bis 80, insbesondere 30 bis 45 Molequivalenten Ethylenoxid bevorzugt sind. Falls das Umhüllungssystem als wäßrige Dispersion auf das Enzymgranulat aufgebracht wird, sind unter den genannten Verbindungen solche mit Ethoxylierungsgraden von 25 bis 50, das heißt Umsetzungsprodukte mit 25 bis 50 Molequivalenten Ethylenoxid, bevorzugt. Alternativ oder zusätzlich zu den Alkoholethoxylaten können auch ethoxylierte Fettsäuren, wobei der Ethoxylierungsgrad vorzugsweise 3 bis 9 beträgt, ethoxylierte Fettsäureamide, wobei der Ethoxylierungsgrad vorzugsweise 4 bis 11 beträgt, und/oder Ethoxylierungsprodukte von Hydroxyfettsäureestern mit 1 bis 6 C-Atomen im Alkoholteil des Esters, beispielsweise Ricinolsäureglycerid, wobei der Ethoxylierungsgrad vorzugsweise 5 bis 80 insbesondere 20 bis 40 beträgt, als Emulgatorkomponente im Überzugssystem eingesetzt werden. Die Fettsäurekomponente der genannten Substanzen besitzt vorzugsweise 12 bis 22 C-Atome. Gewünschtenfalls können die Ethoxygruppen in den genannten Emulgatoren zumindest teilweise durch Propoxygruppen ersetzt sein.

Zu den anorganischen Pigmenten, mit denen eventuelle störende Färbungen des Enzymgranulats überdeckt werden können, gehören beispielsweise Calciumcarbonat, Titandioxid, welches in Rutil- oder Anatase-Kristallmodifikation vorliegen kann, Zinkoxid, Zinksulfid, Bleiweiß (basisches Bleicarbonat), Bariumsulfat, Aluminiumhydroxid, Antimonoxid, Lithopone (Zinksulfid-Bariumsulfat), Kaolin, Kreide und/oder Glimmer. Diese liegen in so feinteiliger Form vor, daß sie in einer Schmelze der übrigen Bestandteile des Überzugssystems oder in Wasser dispergiert werden können. Üblicherweise liegt die mittlere Teilchengröße derartiger Pigmente im Bereich von 0,004 µm bis 50 µm. Insbesondere wenn das Pigment beziehungsweise das gesamte Überzugssystem in Form einer wäßrigen Dispersion eingesetzt werden soll, ist es bevorzugt, daß diese Dispersion Dispergiermittel für das Pigment enthält. Derartige Dispergiermittel können anorganisch, beispielsweise Aluminiumoxid oder Siliziumoxid, welche auch als Pigmente dienen können, oder organisch, beispielsweise Diethylenglykol oder Dipropylenglykol, sein. Der Einsatz mit Dispergiermitteln oberflächenmodifizierter Pigmente ist ebenfalls möglich. Vorzugsweise wird mit Al-, Si-, Zr- oder Polyol-Verbindungen oberflächenmodifiziertes Titandioxidpigment, insbesondere in Rutilform, wie es beispielsweise unter den Handelsnamen Kronos^{(R)} 2132 (Fa. Kronos-Titan) oder Hombitan^{(R)} R 522 (Sachtleben Chemie GmbH) vertrieben wird, eingesetzt. Brauchbar sind auch die Tiona(R) RLL-, AG- bzw. VC-Typen der Fa. Solvay sowie die Bayertitan^{(R)} RD-, R-KB- und AZ-Typen der Fa. Bayer AG.

Als Enzyme kommen in erster Linie die aus Mikroorganismen, wie Bakterien oder Pilzen, gewonnenen Proteasen, Lipasen, Amylasen und/oder Cellulasen in Frage, wobei von Bacillus-Arten erzeugte Proteasen sowie ihre Gemische mit Amylasen bevorzugt sind. Sie werden in bekannter Weise durch Fermentationsprozesse aus geeigneten Mikroorganismen gewonnen, die zum Beispiel in den deutschen Offenlegungsschriften DE 19 40 488, DE 20 44 161, DE 22 01 803 und DE 21 21 397, den US-amerikanischen Patentschriften US 3 632 957 und US 4 264 738 sowie der europäischen Patentanmeldung EP 006 638 beschrieben sind. Besonders vorteilhaft kann das erfindungsgemäße Verfahren zur Formulierung von sehr aktiven Proteasen, die beispielsweise aus der internationalen Patentanmeldung WO 91/02792 bekannt sind, angewendet werden, weil deren lagerstabile Einarbeitung in Wasch- und Reinigungsmittel oft Probleme bereitet und erfindungsgemäß die Entstehung unerwünschter Enzymstäube vermieden wird. Enzyme sind in den erfindungsgemäßen Granulaten vorzugsweise in Mengen von 4 Gew.-% bis 20 Gew.-% enthalten. Falls es sich bei dem erfindungsgemäßen Enzymgranulat um eine proteasehaltige Formulierung handelt, beträgt die Proteaseaktivität vorzugsweise 150 000 Proteaseeinheiten (PE, bestimmt nach der in Tenside 7 (1970), 125 beschriebenen Methode) bis 350 000 PE, insbesondere 160 000 PE bis 300 000 PE, pro Gramm Enzymgranulat.

Als Trägermaterialien für das Enzym sind im Prinzip alle organischen oder anorganischen pulverförmigen Substanzen brauchbar, welche die zu granulierenden Enzyme nicht oder nur tolerierbar wenig zerstören oder desaktivieren und unter Granulationsbedingungen stabil sind. Zu derartigen Substanzen gehören beispielsweise Stärke, Getreidemehl, Cellulosepulver, Alkalialumosilikat, insbesondere Zeolith, Schichtsilikat, zum Beispiel Bentonit oder Smectit, und wasserlösliche anorganische oder organische Salze, zum Beispiel Alkalichlorid, Alkalisulfat, Alkalicarbonat oder Alkaliacetat, wobei Natrium oder Kalium die bevorzugten Alkalimetalle sind. Bevorzugt wird ein Trägermaterialgemisch aus in Wasser quellfähiger Stärke, Getreidemehl und gegebenenfalls Cellulosepulver sowie Alkalicarbonat eingesetzt.

Bei der in Wasser quellfähigen Stärke handelt es sich vorzugsweise um Maisstärke, Reisstärke, Kartoffelstärke oder Gemische aus diesen, wobei der Einsatz von Maisstärke besonders bevorzugt ist. Quellfähige Stärke ist in den erfindungsgemäßen Enzymgranulaten vorzugsweise in Mengen von 20 Gew.-% bis 50 Gew.-%, insbesondere von 25 Gew.-% bis 45 Gew.-% enthalten. Dabei beträgt die Summe der Mengen der quellfähigen Stärke und des Mehls vorzugsweise nicht über 80 Gew.-%, insbesondere 32 Gew.-% bis 65 Gew.-%.

Bei dem Getreidemehl handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird dabei ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten quellfähigen Stärken führenden Getreidearten ist möglich, wenn darauf geachtet wird, daß die Mehle aus den ganzen Körnern hergestellt worden sind. Durch die Mehlkomponente des Zuschlagstoffgemisches wird bekanntermaßen eine wesentliche Geruchsreduzierung der Enzymzubereitung erreicht, welche die Geruchsverminderung durch die Einarbeitung gleicher Mengen entsprechender Stärkearten bei weitem übertrifft. Derartiges Getreidemehl ist in den erfindungsgemäßen Enzymgranulaten vorzugsweise in Mengen von 10 Gew.-% bis 35 Gew.-%, insbesondere von 15 Gew.-% bis 25 Gew.-% enthalten.

Die erfindungsgemäßen Enzymgranulate enthalten als weitere Komponente des Trägermaterials vorzugsweise 1 Gew.-% bis 50 Gew.-%, vorzugsweise 5 Gew.-% bis 25 Gew.-%, bezogen auf gesamtes Granulat, eines Granulierhilfsmittelsystems, das Alkali-Carboxymethylcellulose mit Substitutionsgraden von 0,5 bis 1 und Polyethylenglykol und/oder Alkylpolyethoxylat enthält. In diesem Granulierhilfsmittelsystem sind vorzugsweise, jeweils bezogen auf fertiges Enzymgranulat, 0,5 Gew.-% bis 5 Gew.-% Alkali-Carboxymethylcellulose mit Substitutionsgraden von 0,5 bis 1 und bis zu 3 Gew.-% Polyethylenglykol und/oder Alkylpolyethoxylat enthalten, wobei besonders bevorzugt ist, wenn mindestens 0,5 Gew.-%, insbesondere 0,8 Gew.-% bis 2 Gew.-% Polyethylenglykol mit einer mittleren Molmasse unter 1000 und/oder Alkylpolyethoxylat mit mindestens 30 Ethoxygruppen vorhanden ist, falls mehr als 2 Gew.-% Alkali-Carboxymethylcellulose enthalten ist. Höher substituierte Carboxymethylcellulose, mit Substitutionsgraden bis zu 3, ist in dem Granulierhilfsmittelsystem vorzugsweise nicht enthalten.

Gegebenenfalls können als zusätzliche Bestandteile des Granulierhilfsmittel systems auch weitere Cellulose- oder Stärkeether, wie Carboxymethylstärke, Methylcellulose, Hydroxyethylcellulose, Hydroxypropylcellulose sowie entsprechende Cellulosemischether, Gelatine, Casein, Traganth, Maltodextrose, Saccharose, Invertzucker, Glukosesirup oder andere in Wasser lösliche beziehungsweise gut dispergierbare Oligomere oder Polymere natürlichen oder synthetischen Ursprungs verwendet werden. Brauchbare synthetische wasserlösliche Polymere sind Polyacrylate, Polymethacrylate, Copolymere der Acrylsäure mit Maleinsäure oder vinylgruppenhaltige Verbindungen, ferner Polyvinylalkohol, teilverseiftes Polyvinylacetat und Polyvinylpyrrolidon. Soweit es sich bei den vorgenannten Verbindungen um solche mit freien Carboxylgruppen handelt, liegen sie normalerweise in Form ihrer Alkalisalze, insbesondere ihrer Natriumsalze vor. Derartige zusätzliche Granulierhilfsmittel können in den erfindungsgemäßen Enzymgranulaten in Mengen bis zu 10 Gew.-%, insbesondere von 0,5 Gew.-% bis 8 Gew.-% enthalten sein. Höhermolekulare Polyethylenglykole, das heißt solche mit einem mittleren Molekulargewicht über 1000, sind zwar als synthetische wasserlösliche Polymere mit staubbindender Wirkung brauchbar, doch bewirken gerade die höhermolekularen Polyethylenglykole eine unerwünschte Erhöhung der benötigten Granulatauflösezeit, so daß diese Substanzen in den erfindungsgemäßen Enzymgranulaten vorzugsweise völlig fehlen.

Zur Herstellung der erfindungsgemäßen Enzymgranulate geht man vorzugsweise von Fermentbrühen aus, die beispielsweise durch Mikrofiltration von unlöslichen Begleitstoffen befreit werden. Die Mikrofiltration wird dabei vorzugsweise als Querstrom-Mikrofiltration unter Verwendung poröser Rohre mit Mikroporen größer 0,1 um, Fließgeschwindigkeiten der Konzentratlösung von mehr als 2 m/s und einem Druckunterschied zur Permeatseite von unter 5 bar durchgeführt, wie beispielsweise in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration kann dabei, wie in der internationalen Patentanmeldung WO 92/11347 beschrieben, so geführt werden, daß man nur zu relativ niedrigen Gehalten an Trockensubstanz von vorzugsweise 5 Gew.-% bis 50 Gew.-%, insbesondere von 10 Gew.-% bis 40 Gew.-% gelangt. Das Konzentrat wird einem zweckmäßigerweise zuvor hergestellten trockenen, pulverförmigen bis körnigen Gemisch der oben beschriebenen Zuschlagstoffe zudosiert. Der Wassergehalt der Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Das rieselfähige Vorgemisch wird im Prinzip bekannter Weise anschließend in einem Kneter sowie einem angeschlossenen Extruder zu einer plastischen, möglichst homogenen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,7 mm bis 1,2 mm, vorzugsweise 0,8 mm bis 1,0 mm. Die in dieser Form vorliegenden Partikel können anschließend getrocknet und mit dem Überzugssystem gemäß der Erfindung umhüllt werden. Es hat sich jedoch als vorteilhaft erwiesen, die den Extruder und Zerhacker verlassenden zylindrischen Partikel vor dem Umhüllen zu sphäronisieren, das heißt sie in geeigneten Vorrichtungen abzurunden und zu entgraten. Man verwendet hierzu eine Vorrichtung, die aus einem zylindrischen Behälter mit stationären, festen Seitenwänden und einer bodenseitig drehbar gelagerten Reibplatte bestehen. Vorrichtungen dieser Art sind unter der Warenbezeichnung Marumerizer^{(R)} in der Technik verbreitet und beispielsweise in den deutschen Auslegeschriften DE 21 37 042 und DE 21 37 043 beschrieben. Anschließend können eventuell auftretende staubförmige Anteile mit einer Korngröße unter 0,1 mm, insbesondere unter 0,4 mm sowie eventuelle Grobanteile mit einer Korngröße über 2 mm, insbesondere über 1,6 mm durch Sieben oder Windsichten entfernt und gegebenenfalls in den Herstellungsprozess zurückgeführt werden. Nach der Sphäronisierung werden die Kügelchen kontinuierlich oder chargenweise, vorzugsweise unter Verwendung einer Wirbelschichttrockenanlage, bei Zulufttemperaturen von vorzugsweise 35 °C bis 50 °C und insbesondere bei einer Produkttemperatur von nicht über 42 °C bis zum gewünschten Restfeuchtegehalt von beispielsweise 4 Gew.-% bis 10 Gew.-%, insbesondere 5 Gew.-% bis 8 Gew.-%, bezogen auf gesamtes Granulat, getrocknet.

Nach oder vorzugsweise während der Trocknung wird das Überzugssystem gemäß der Erfindung als äußere Umhüllung aufgebracht. In einer Ausgestaltung des erfindungsgemäßen Herstellungsverfahrens wird das Überzugssystem als wäßrige Dispersion, die vorzugsweise 50 Gew.-% bis 70 Gew.-% Wasser und 30 Gew.-% bis 50 Gew.-% des Überzugssystems enthält, wobei im Überzugssystem insbesondere 1 Gew.-% bis 2,5 Gew.-%, bezogen auf gesamtes Überzugssystem, Dispergiermittel für das Pigment enthalten ist, in die Wirbelschicht aus Extrudat eingebracht. Das über die wäßrige Dispersion zugeführte Wasser wird bei der gleichzeitig vorgenommenen oder einer anschließend erneut erforderlichen Trocknung wieder entfernt. In einer weiteren Ausgestaltung des erfindungsgemäßen Herstellungsverfahrens wird das Überzugssystem, gegebenenfalls unter Kühlung, als erwärmte, bei einer Temperatur von 5 °C bis 45 °C über dem Schmelzpunkt der Alkoholkomponente vorliegende Flüssigkeit auf das Extrudat aufgebracht. Außerdem ist eine Kombination dieser Vorgehensweisen, die darin besteht, einen Teil des Überzugssystems in Form einer wäßrigen Dispersion und einen zweiten Teil als Schmelze aufzubringen, möglich. Vorzugsweise bringt man, bezogen auf fertiges Granulat, 6 Gew.-% bis 15 Gew.-% des Überzugssystems als äußere Umhüllungsschicht auf das enzymhaltige Extrudat auf.

Die nach dem erfindungsgemäßen Verfahren erhaltene Enzymzubereitung besteht aus weitgehend abgerundeten, gleichmäßig umhüllten und staubfreien Partikeln, die in der Regel ein Schüttgewicht von etwa 500 bis 900 Gramm pro Liter, insbesondere 650 bis 880 Gramm pro Liter aufweisen. Die erfindungsgemäßen Granulate zeichnen sich durch eine sehr hohe Lagerstabilität, insbesondere bei Temperaturen über Raumtemperatur und hoher Luftfeuchtigkeit, sowie ein rasches Lösungsverhalten in der Waschflotte aus. Vorzugsweise setzen die erfindungsgemäßen Granulate 100 % ihrer Enzymaktivität innerhalb von 3 Minuten, insbesondere innerhalb von 90 Sekunden bis 2 Minuten, in Wasser bei 25 °C frei.

Das erfindungsgemäße oder nach dem erfindungsgemäßen Verfahren hergestellte Enzymgranulat wird vorzugsweise zur Herstellung fester, insbesondere teilchenförmiger Wasch- oder Reinigungsmittel verwendet, die durch einfaches Vermischen der Enzymgranulate mit in derartigen Mitteln üblichen weiteren Pulverkomponenten erhalten werden können. Für die Einarbeitung in teilchenförmige Wasch- und Reinigungsmittel weist das Enzymgranulat vorzugsweise mittlere Korngrößen im Bereich von 0,8 mm bis 1,2 mm auf. Die erfindungsgemäßen Granulate enthalten vorzugsweise weniger als 2 Gew.-%, insbesondere höchstens 1,4 Gew.-% an Partikeln mit Korngrößen außerhalb des Bereichs von 0,4 mm bis 1,6 mm.

### Beispiele

### Beispiel 1

Ein nach der Fermentation gewonnener Erntebrei, wie in der internationalen Patentanmeldung WO 91/02792 beschrieben, mit 75 000 Proteaseeinheiten pro g (PE/g) wurde nach der Entfernung der Fermentationsrückstände durch Dekantieren und Mikrofiltration in einer Ultrafiltrationsanlage aufkonzentriert. Nach der weiteren Aufkonzentrierung mittels Vakuumeindampfung enthielt die wäßrige Enzymsuspension 700 000 PE/g. Dieses Proteasekonzentrat wurde mit Zuschlagstoffen (3,5 Gew.-% Saccharose, 4,5 Gew.-% Cellulose, 3 Gew.-% Carboxymethylcellulose mit Substitutionsgrad 0,65-0,75, 19 Gew.-% Weizenmehl, 35 Gew.-% Maisstärke und 3 Gew.-% Polyethylenglykol, jeweils bezogen auf entstehendes Gemisch) vermischt, homogenisiert und anschließend in einem Extruder mit Schneidevorrichtung in Granulate überführt. Der Lochdurchmesser der Lochplatte des Extruders betrug 0,9 mm. Das Verhältnis von Länge zu Dicke des Granulatkorns lag bei 1. Nach der Verrundung und Trocknung der Granulate wurden die Partikel mit Teilchengröße kleiner 0,4 mm und größer 1,6 mm abgesiebt. Die Kornfraktion zwischen 0,4 mm und 1,6 mm wurde in einem Wirbelschicht-Sprühgranulator des Typs STREA-1 der Fa. Aeromatic in der Wirbelschicht gecoated. Während des Coatens wurden folgende Betriebsparameter eingestellt:

| | |
|---|---|
| Zulufttemperatur | 47 °C |
| Produkttemperatur | 36 °C |
| Ablufttemperatur | 33 °C |
| Luftmenge | 90 m³/h |
| Durchsatzrate an Coatingssupension | 8 g/min |

Die Coatingssuspension bestand aus:

| | |
|---|---|
| Titandioxid | 17 % |
| technischem Stearylalkohol | 19 % |
| Eumulgin^{(R)} RT 40^{a)} | 3 % |
| Wasser | 61 % |

| | |
|---|---|
| ^{a)} 40-fach ethoxyliertes Ricinusöl, Hersteller Henkel KGaA | |

Der Stearylalkohol besaß die folgende C-Kettenverteilung: C₁₆ 0 - 5 %, C₁₈ 95 - 100 %, C₂₀ 0 - 2 %, eine Hydroxylzahl von 203 - 210 und einen Erstarrungsbereich von 55 - 57,5 °C.

Zur Herstellung der Coatingssuspension wurde das Wasser auf ca. 70 °C temperiert und mit dem flüssigen Emulgator vermischt. Der in fester Form vorliegende Stearylalkohol wurde in die Wasser/Emulgator-Lösung eingerührt und dabei aufgeschmolzen. Nach Zugabe des Titandioxids lag eine homogene Emulsion vor, in der das Titandioxidpigment gleichmäßig ohne Agglomeratbildung verteilt war. Diese Coatingsuspension wurde bei den oben angegebenen Betriebsparametern auf das Enzymextrudat aufgesprüht. Das Wasser der Coatingsuspension verdampfte und wurde mit der Abluft ausgetragen. Nach Aufsprühen von etwa 285 g Coatingsuspension pro kg Enzymgranulat waren die Extrudate gleichmäßig mit einer weißen Farb- und Schutzschicht umhüllt. Der Fettalkohol bildete auf der Granulatoberfläche einen gleichmäßigen, unporösen Film aus.

Zur Bestimmung des Staubabriebes wurden 60 g Granulat in eine Wirbelschicht gegeben. Die Abluft der Wirbelschicht strömte durch einen Filter. Die nach 40 Minuten Verweilzeit des Enzymgranulats unter diesen Bedingungen aufgefangene Staubmenge entspricht der Staubabriebmenge. Im vorliegenden Fall war der Staubabrieb mit ≤ 0,04 Gew.-% vernachlässigbar gering.

### Beispiel 2:

Es wurde wie in Beispiel 1 gearbeitet. Allerdings wurden nur 150 g Coatingsuspension pro kg Enzymgranulat aufgesprüht. Die endgültige weiße Farbgebung erfolgte in einem zweiten Coatingschritt. Das vorgefärbte Enzymgranulat wurde in einem Rotorgranulator des Typs GPCG-5 der Fa. Glatt mit einer zweiten Coatingschicht überzogen. In der zweiten Coatingstufe wurde statt mit einer wäßrigen Coatingsuspension mit einer Schmelze gearbeitet. Die Coatingschmelze hatte die gleichen Inhaltsstoffe wie die wäßrige Pigmentsuspension gemäß Beispiel 1 mit Ausnahme des Wassers. Der Stearylalkohol wurde aufgeschmolzen und mit Emulgator und Titandioxid unter Rühren vermischt. 8 Gew.-%, bezogen auf entstehendes Enzymgranulat, der Schmelze wurden in dieser zweiten Coatingstufe mit 100 °C auf das vorgecoatete Enzymgranulat unter folgenden Betriebsbedingungen aufgesprüht:

| | |
|---|---|
| Zulufttemperatur | 44 °C |
| Produkttemperatur | 41 °C |
| Ablufttemperatur | 41 °C |
| Luftmenge | 75 m³/h |
| Durchsatzrate an Coatingschmelze | 12 g/min. |

Das in 2 Stufen gecoatete Produkt hatte einen höheren Weißgrad bei der gleichen Menge an Titandioxid wie im einstufigen Verfahren gemäß Beispiel 1. Der Staubabrieb war vergleichbar gering. Das Produkt ist wie das Enzymgranulat gemäß Beispiel 1 nahezu geruchsneutral.

### Beispiele 3 und 4:

Es wurde wie in den Beispielen 1 beziehungsweise 2 gearbeitet. Allerdings wurde in der Coatingschmelze statt des flüssigen ethoxylierten Ricinusöls ein festes Fettalkoholethoxylat (Lutensol^{(R)} AT 80, Hersteller BASF) mit folgenden Kenndaten verwendet:

| | |
|---|---|
| Ethoxylierungsgrad | 80 |
| Trübungspunkt | 100 °C |
| Molare Masse | 3780 |
| Tropfpunkt | 56 °C |
| Erstarrungspunkt | 42 °C |
| Viskosität bei 60°C | 400 mPas |
| Hydroxylzahl | 14 |
| HLB-Wert | 18,5. |

Die Eigenschaften der so hergestellten Enzymgranulate unterschieden sich nicht signifikant von denen gemäß Beispiel 1 beziehungsweise 2.

## Patentansprüche

1. Für die Einarbeitung in Wasch- oder Reinigungsmittel geeignetes Enzymgranulat, enthaltend Enzym und anorganisches und/oder organisches Trägermaterial und eine gleichmäßige äußere pigmenthaltige Umhüllungsschicht, dadurch gekennzeichnet, daß die äußere Umhüllungsschicht aus einem Überzugssystem besteht, welches 30 Gew.-% bis 50 Gew.-% feinteiliges anorganisches Pigment, 45 Gew.-% bis 60 Gew.-% eines Alkohols oder Alkoholgemisches mit einem Schmelzpunkt im Bereich von 45 °C bis 65 °C, bis zu 15 Gew.-% Emulgator für den Alkohol, bis zu 5 Gew.-% Dispergiermittel für das Pigment und bis zu 3 Gew.-% Wasser enthält.

2. Enzymgranulat nach Anspruch 1, dadurch gekennzeichnet, daß es 5 Gew.-% bis 15 Gew.-% Emulgator für den Alkohol und/oder 0,2 Gew.-% bis 3 Gew.-% Dispergiermittel für das Pigment enthält.

3. Enzymgranulat nach Anspruch 1 oder 2, dadurch gekennzeichnet, daß die Umhüllungsschicht als anorganisches Pigment Titandioxid, Zinkoxid, Kreide und/oder Calciumcarbonat enthält.

4. Enzymgranulat nach einem der Ansprüche 1 bis 3, dadurch gekennzeichnet, daß der Alkohol in der Umhüllungsschicht ein primärer linearer Alkohol mit 14 bis 22 C-Atomen oder ein Gemisch aus diesen ist.

5. Enzymgranulat nach einem der Ansprüche 1 bis 4, dadurch gekennzeichnet, daß der Emulgator für den Alkohol ein mit durchschnittlich 25 bis 50, insbesondere 30 bis 45 Molequivalenten Ethylenoxid veretherter primärer linearer gesättigter oder ungesättigter Alkohol mit 14 bis 22 C-Atomen, eine ethoxylierte Fettsäure, wobei der Ethoxylierungsgrad insbesondere 3 bis 9 beträgt, ein ethoxyliertes Fettsäureamid, wobei der Ethoxylierungsgrad insbesondere 4 bis 11 beträgt, ein Ethoxylierungsprodukt von Hydroxyfettsäureestern mit 1 bis 6 C-Atomen im Alkoholteil des Esters, wobei der Ethoxylierungsgrad vorzugsweise 5 bis 80 beträgt, oder ein Gemisch aus diesen ist.

6. Enzymgranulat nach einem der Ansprüche 1 bis 5, dadurch gekennzeichnet, daß es eine mittlere Korngröße von 0,8 mm bis 1,2 mm aufweist und weniger als 2 Gew.-%, insbesondere höchstens 1,4 Gew.-% an Partikeln mit Korngrößen außerhalb des Bereichs von 0,4 mm bis 1,6 mm enthält.

7. Enzymgranulat nach einem der Ansprüche 1 bis 6, dadurch gekennzeichnet, daß es Protease, Amylase, Lipase und/oder Cellulase enthält.

8. Enzymgranulat nach einem der Ansprüche 1 bis 7, dadurch gekennzeichnet, daß es Protease mit einer Aktivität von 150 000 PE bis 350 000 PE, insbesondere 160 000 PE bis 300 000 PE, pro Gramm Enzymgranulat enthält.

9. Verfahren zur Herstellung eines für die Einarbeitung in Wasch- oder Reinigungsmittel geeigneten Enzymgranulates durch Extrudieren eines durch Vermischen einer aufkonzentrierten Fermentationsbrühe mit anorganischem und/oder organischem Trägermaterial als Zuschlagstoff entstandenen Enzym-Vorgemischs, gegebenenfalls Sphäronisierung des Extrudats in einem Rondiergerät, Trocknung und Aufbringen einer äußeren Umhüllungsschicht, dadurch gekennzeichnet, daß man in einer Wirbelschicht aus Extrudat eine äußere Umhüllungsschicht eines Überzugssystems aus 30 Gew.-% bis 50 Gew.-% feinteiligem anorganischem Pigment, 45 Gew.-% bis 60 Gew.-% Alkohol mit einem Schmelzpunkt im Bereich von 45 °C bis 65 °C, bis zu 15 Gew.-%, insbesondere 5 Gew.-% bis 15 Gew.-% Emulgator für den Alkohol und bis zu 5 Gew.-%, insbesondere 0,2 Gew.-% bis 3 Gew.-% Dispergiermittel für das Pigment aufbringt.

10. Verfahren nach Anspruch 9, dadurch gekennzeichnet, daß man, bezogen auf fertiges Granulat, 6 Gew.-% bis 15 Gew.-% des Überzugssystems als äußere Umhüllungsschicht auf das enzymhaltige Extrudat aufbringt.

11. Verfahren nach Anspruch 9 oder 10, dadurch gekennzeichnet, daß man die Umhüllungsschicht als wäßrige Dispersion ihrer Bestandteile auf das enzymhaltige Extrudat aufbringt und gleichzeitig und/oder anschließend das Wasser durch Trocknen bei einer Temperatur, die das Enzymgranulat auf nicht über 42 °C erwärmt, entfernt.

12. Verfahren nach Anspruch 11, dadurch gekennzeichnet, daß man eine Dispersion einsetzt, die 50 Gew.-% bis 70 Gew.-% Wasser und 30 Gew.-% bis 50 Gew.-% des Überzugssystems enthält, wobei im Überzugssystem 1 Gew.-% bis 2,5 Gew.-%, bezogen auf gesamtes Überzugssystem, Dispergiermittel für das Pigment enthalten ist.

13. Verfahren nach einem der Ansprüche 9 bis 12, dadurch gekennzeichnet, daß man das Überzugssystem als Flüssigkeit, die bei einer Temperatur von 5 °C bis 45 °C über dem Schmelzpunkt der Alkoholkomponente vorliegt, auf das Extrudat aufbringt.

14. Verfahren nach einem der Ansprüche 9 bis 13, dadurch gekennzeichnet, daß man nach der Sphäronisierung des Extrudats das Granulat bei Temperaturen von 35 °C bis 50 °C auf einen Wassergehalt von 4 Gew.-% bis 10 Gew.-% trocknet.

15. Verwendung eines Enzymgranulats gemäß einem der Ansprüche 1 bis 8 zur Herstellung fester, insbesondere teilchenförmiger Wasch- oder Reinigungsmittel.

## Claims

1. Enzyme granules suitable for incorporation in detergents or cleaning compositions containing enzyme and inorganic and/or organic carrier material and a uniform outer pigment-containing coating layer, characterized in that the outer coating layer consists of a coating system containing 30 to 50% by weight of fine-particle inorganic pigment, 45 to 60% by weight of an alcohol or alcohol mixture with a melting point in the range from 45°C to 65°C, up to 15% by weight of an emulsifier for the alcohol, up to 5% by weight of dispersant for the pigment and up to 3% by weight of water.

2. Enzyme granules as claimed in claim 1, characterized in that they contain 5% by weight to 15% by weight of emulsifier for the alcohol and/or 0.2% by weight to 3% by weight of dispersant for the pigment.

3. Enzyme granules as claimed in claim 1 or 2, characterized in that the coating layer contains titanium dioxide, zinc oxide, chalk and/or calcium carbonate as the inorganic pigment.

4. Enzyme granules as claimed in any of claims 1 to 3, characterized in that the alcohol in the coating layer is a primary linear alcohol containing 14 to 22 carbon atoms or a mixture thereof.

5. Enzyme granules as claimed in any of claims 1 to 4, characterized in that the emulsifier for the alcohol is a primary, linear, saturated or unsaturated C₁₄₋₂₂ alcohol etherified with on average 25 to 50 mole equivalents and, more particularly, with 30 to 45 mole equivalents of ethylene oxide, an ethoxylated fatty acid with a degree of ethoxylation of, in particular, 3 to 9, an ethoxylated fatty acid amide with a degree of ethoxylation of, in particular, 4 to 11, an ethoxylation product of hydroxyfatty acid esters containing 1 to 6 carbon atoms in the alcohol part of the ester, the degree of ethoxylation preferably being from 5 to 80, or a mixture thereof.

6. Enzyme granules as claimed in any of claims 1 to 5, characterized in that they have an average particle size of 0.8 mm to 1.2 mm and contain less than 2% by weight and, more particularly, at most 1.4% by weight of particles with sizes outside the 0.4 mm to 1.6 mm range.

7. Enzyme granules as claimed in any of claims 1 to 6, characterized in that they contain protease, amylase, lipase and/or cellulase.

8. Enzyme granules as claimed in any of claims 1 to 7, characterized in that they contain protease with an activity of 150,000 PU to 350,000 PU and, more particularly, 160,000 PU to 300,000 PU per gram of enzyme granules.

9. A process for the production of enzyme granules suitable for incorporation in detergents or cleaning compositions by extruding an enzyme compound formed by mixing of a concentrated fermentation broth with inorganic and/or organic carrier material as additive, optionally spheronizing the extrudate in a spheronizer, drying and applying an outer coating layer, characterized in that an outer coating layer of a coating system of 30% by weight to 50% by weight of fine-particle inorganic pigment, 45% by weight to 60% by weight of alcohol with a melting point of 45°C to 65°C, up to 15% by weight and, more particularly, 5% by weight to 15% by weight of emulsifier for the alcohol and up to 5% by weight and, more particularly, 0.2% by weight to 3% by weight of dispersant for the pigment is applied in a fluidized bed of extrudate.

10. A process as claimed in claim 9, characterized in that 6% by weight to 15% by weight, based on the final granules, of the coating system is applied to the enzyme-containing extrudate as an outer coating layer.

11. A process as claimed in claim 9 or 10, characterized in that the coating layer is applied to the enzyme-containing extrudate as an aqueous dispersion of its constituents and the water is removed at the same time and/or subsequently by drying at a temperature which heats the enzyme granules to a temperature of not more than 42°C.

12. A process as claimed in claim 11, characterized in that a dispersion containing 50% by weight to 70% by weight of water and 30% by weight to 50% by weight of the coating system is used, the coating system containing 1% by weight to 2.5% by weight, based on the coating system as a whole, of dispersant for the pigment.

13. A process as claimed in any of claims 9 to 12, characterized in that the coating system is applied to the extrudate as a liquid which is present at a temperature of 5°C to 45°C above the melting point of the alcohol component.

14. A process as claimed in any of claims 9 to 13, characterized in that, after spheronizing of the extrudate, the granules are dried to a water content of 4% by weight to 10% by weight at temperatures of 35°C to 50°C.

15. The use of the enzyme granules claimed in any of claims 1 to 8 for the production of solid, more particularly particulate, detergents or cleaning compositions.

## Revendications

1. Granulé d'enzyme approprié pour l'incorporation dans les produits de lavage ou de nettoyage, contenant un enzyme et un matériau de support minéral et/ou organique et une couche d'enrobage régulière externe contenant un pigment,
**caractérisé en ce que**
la couche d'enrobage externe consiste en un système de revêtement, qui contient de 30 % en poids à 50 % en poids de pigment minéral finement divisé ; de 45 % en poids à 60 % en poids d'un alcool ou d'un mélange d'alcools ayant un point de fusion dans la plage de 45°C à 65°C, jusqu'à 15 % en poids d'un agent émulsionnant pour l'alcool, jusqu'à 5 % en poids d'agent dispersant pour le pigment et jusqu'a 3 % en poids d'eau.

2. Granulé d'enzyme selon la revendication 1,
caractérisé en ce qu'
il renferme de 5 % en poids à 15 % en poids d'agent émulsionnant pour l'alcool et/ou 0,2 % en poids à 3 % en poids d'agent dispersant pour le pigment.

3. Granulé d'enzyme selon la revendication 1 ou la revendication 2,
caractérisé en ce que
la couche d'enrobage renferme comme pigment minéral, du dioxyde de titane, de l'oxyde de zinc, de la craie et/ou du carbonate de calcium.

4. Granulé d'enzyme selon l'une des revendications 1 à 3,
caractérisé en ce que
l'alcool dans la couche d'enrobage est un alcool primaire linéaire ayant de 14 à 22 atomes de carbone ou un mélange à base de ceux-ci.

5. Granulé d'enzyme selon l'une des revendications 1 à 4,
**caractérisé en ce que**
l'agent émulsionnant pour l'alcool est un alcool primaire, linéaire, saturé ou non saturé, éthérifié avec en moyenne 25 à 50, en particulier 30 à 45 équivalents molaires d'oxyde d'éthylène, ayant de 14 à 22 atomes de carbone, un *acide* gras éthoxylé dans lequel le degré d'éthoxylation s'élève en particulier de 3 à 9, un amide d'acide gras éthoxylé dans lequel le degré d'éthoxylation s'élève en particulier de 4 à 11, un produit d'éthoxylation d'esters d'acide gras hydroxylé ayant de 1 à 6 atomes de carbone dans la partie alcool de l'ester dans lequel le degré d'éthoxylation s'élève de préférence de 5 à 80 ou est un mélange à base de ceux-ci.

6. Granulé d'enzyme selon l'une des revendications 1 à 56,
caractérisé en ce qu'
il possède une taille moyenne de grains allant de 0,8 mm à 1,2 mm, et renferme moins de 2 % en poids, en particulier au maximum 1,4 % en poids, de particules ayant des tailles de grain en dehors de la zone de 0,4 mm à 1,6 mm.

7. Granulé d'enzyme selon l'une des revendications 1 à 6,
caractérisé en ce qu'
il renferme de la protéase, de l'amylase, de la lipase et/ou de la cellulase.

8. Granulé d'enzyme selon l'une des revendications 1 à 7,
caractérisé en ce qu'
il renferme de la protéase ayant une activité de 150.000 PE à 350.000 PE, en particulier de 160.000 PE à 300.000 PE par gramme de granulé d'enzyme.

9. Procédé de production d'un granulé d'enzyme approprié pour l'incorporation dans des produits de lavage ou de nettoyage par extrusion d'un pré-mélange d'enzyme formé par mélange d'un bouillon de fermentation concentré, avec un matériau de support minéral et/ou organique comme substance d'addition, le cas échéant par sphéronisation de l'extrudat dans un appareil pour arrondir, séchage et application d'une couche d'enrobage externe,
caractérisé en ce qu'
on applique dans une couche fluidisée à partir de l'extrudat, une couche d'enrobage externe d'un système de revêtement à base de 30 % en poids à 50 % en poids de pigment minéral finement divisé, de 45 % en poids à 60 % en poids d'un alcool ayant un point de fusion dans la plage de 45 à 65°C, jusqu'à 15 % en poids, en particulier de 5 % en poids à 15 % en poids d'un agent émulsionnant pour l'alcool, en jusqu'à 5 % en poids, en particulier de 0,2 % en poids à 3 % en poids d'agent dispersant pour le pigment.

10. Procédé selon la revendication 9,
**caractérisé en ce qu'**
on applique de 6 % en poids à 15 % en poids, rapporté au granulé terminé, du système de revêtement en tant que couche d'enrobage externe, sur l'extrudat contenant l'enzyme.

11. Procédé selon la revendication 9 ou 10,
**caractérisé en ce qu'**
on applique la couche d'enrobage sous forme de dispersion aqueuse de leurs constituants sur l'extrudat contenant l'enzyme et en même temps et/ou successivement, on élimine l'eau par séchage à une température, qui échauffe le granule d'enzyme à pas plus que 42°C.

12. Procédé selon la revendication 11,
**caractérisé en ce qu'**
on met en oeuvre une dispersion qui contient de 50 % en poids à 70 % en poids d'eau et de 30 % en poids à 50 % en poids du système de revêtement, tandis que dans le système de revêtement 1% en poids à 2,5 % en poids, rapporté au système de revêtement total, d'agent de dispersion pour le pigment, est contenu.

13. Procédé selon l'une des revendications 9 à 12,
caractérisé en ce qu'
on applique sur l'extrudat le système de revêtement sous forme de liquide qui se présente à une température de 5°C à 45°C au-dessus du point de fusion du composant alcool.

14. Procédé selon l'une des revendications 9 à 13,
caractérisé en ce qu'
on sèche après la sphéronisation de l'extrudat, le granulé à des températures de 35°C à 50°C, à une teneur en eau allant de 4 % en poids à 10 % en poids.

15. Utilisation d'un granulé d'enzyme conformément à l'une des revendications 1 à 8, en vue de la production de produits de lavage ou de nettoyage solides, en particulier sous forme de particules.
